# EUROPEAN PATENT APPLICATION

(11) **EP 4 744 674 A1**
(43) Date of publication of application: **20.05.2026**
(21) Application number: 24843219.7
(22) Date of filing: 31.01.2024
(51) Int. Cl.: A61K 35/747, A61P 1/00, A23L 33/135, A23L 25/00, A23L 29/10, A23L 27/10, A23L 29/30, A23L 29/20, A23L 29/00

(54) **GASTRIC ACID-STABLE PROBIOTIC PREPARATION AND METHOD FOR PREPARING SAME**

(30) Priority: 14.07.2023 KR 20230091984
(71) Applicant: Valvet Care Co., Ltd., Seoul 06794 (KR)
(72) Inventor: KIM, Yong Min, Seoul 04401 (KR); JEON, Ju Hyun, Seoul 06106 (KR)
(74) Representative: Viering, Jentschura & Partner mbB Patent- und Rechtsanwälte
(86) International application number: PCT/KR2024/001495
(87) International publication number: WO 2025/018492

(57) **Abstract**

A gastric acid-stable probiotic formulation strongly exerts an excellent and advanced effect than the previously known lactic acid bacteria preparation through various experiments, for examples, (1) experiment for the preparation of emulsion by heating temperature (Experimental Example 1); (2) Effect on the proliferation of beneficial bacteria in the intestine (Experimental Example 2); (3) Lactobacillus viability experiment in artificial gastric juice (pH 2) (Experimental Example 3); (4) Efficacy test of composition for improving constipation syndrome (simple clinical test 1); (5) palatability evaluation experiment according to the type of nuts contained in the composition (simple clinical test 2), and found the inventive composition strongly proliferates beneficial bacteria in the intestine, exhibits excellent viability of lactic acid bacteria in stomach acid, and exerts an excellent and advanced effect in terms of improvement of constipation and taste than the previously known lactic acid bacteria preparation.

## Description

### CROSS REFERENCE TO RELATED APPLICATIONS

This application claims priority to Korean Patent Application No. 10-2023-0091984 filed on July 14, 2023, which is hereby incorporated by reference in its entirety.

### BACKGROUND OF THE INVENTION

The present invention relates to a gastric acid-stable probiotic formulation containing edible oils, nuts and emulsifiers, and the method thereof.

Generally, lactic acid bacteria settle in the intestine and exert various bioactive effects such as activating intestinal motility, suppressing harmful bacteria, and promoting vitamins and immune enhancing substances.

For this reason, there have been attempts to prevent the death of lactic acid bacteria in the body by coating lactobacillus powder with various proteins or carbohydrates, and accordingly, lactic acid bacteria powder coated with various substances and a method for preparing the same have been developed.

There are about 400 kinds of bacteria present in the human colon and feces, and more than 90% of them are anaerobic strains. They maintain a certain balance and form a microflora in the intestines, and are affected by the health status of the human body, stress, and physiological changes of aging, as well as by food and drugs introduced from the outside.. For physical health and life extension, it is necessary to maintain more beneficial bacteria in the intestinal flora and less harmful bacteria.

In 2001, a joint expert committee of the World Health Organization and the World Food Organization defined "probiotics" as live microorganisms that are beneficial to health when consumed in appropriate amounts (Hye Mi Jeong et al., 2011, J Korean Soc. Food Sci. Nutr. 40(4), pp.500-508). This refers to probiotics that can help improve the gut microbiome. On the other hand, prebiotics refer to substances that can be used by useful microorganisms in the intestine and can help the growth and function of beneficial bacteria in the intestine. Non-pulsated polysaccharides and oligosaccharides, which are not easily broken down by human enzymes but can be utilized by microorganisms, have been known as prebiotics till now (Manning TS. Gibson GR. 2004. Prebiotics. Best Practice & Research Clinical Gastroenterology. 18(2): pp.287-298). Recently, the effects of polyphenols, which can have a beneficial effect on the intestinal microbiota, have become known, and the concept of these prebiotics has been broadened (Cardona et al., 2013, Journal of Nutritional biochemistry, 24, pp1415-1422).

On the other hand, lactic acid bacteria are representative bacteria that are widely used as probiotics, and they are microorganisms that are widely present in nature and are easily found in the intestines of humans and animals and in fermented foods, and are recognized as safe by the U.S. Food and Drug Administration. Lactic acid bacteria attach to intestinal epithelial cells and parasitize them, thereby improving the properties of intestinal flora, stabilizing intestinal flora, reducing the production of decay products by inhibiting the settlement of harmful bacteria, resulting in various beneficial advantages to host animals, such as prevention of various diseases, immune activation, anti-cancer effects, reducing cholesterol level etc. The reason why lactic acid bacteria have a growth-inhibiting effect on various putrefactive and pathogenic microorganisms is due to several metabolic properties, such as organic acid, hydrogen peroxide, reuterin, diacetyl, acetaldehyde, and bacteriocin as metabolites (Fuller, K., 1989, Probiotics in man and animals, J. Appl. Bacteriol., 66, pp.365-378; Korea Patent publication No. 10-2015-0075447 A)

Probiotics are currently mainly used as health functional food, but they are also used for therapeutic purposes, for example, there have been several attempts to develops several treating agents, specifically, it increases the production of immunoglobulin A antibodies and reduces rotavirus shedding to shorten the prevalence of diarrhea, so it is actively being applied as an adjuvant for the prevention and treatment of antibiotic-associated diarrhea, inflammatory bowel diseases such as ulcerative colitis or Crohn's disease, abdominal bloating or constipation, irritable colitis, radiation-induced enteritis, and Helicobacter infection treatment adjuvant. Particularly, it is reported to be effective as a treatment for chronic inflammatory bowel disease (Korea Patent publication No. 10-2008-0075971 A).

On the other hand, the prior patent literature is as follows.

Korea Patent publication No. 10-2022-0068518 A (Prior art 3, Applicant name: VALVET CARE CO., LTD.) discloses a composition for inducing voluntary solid preparation intake by a pet. The composition contains unsaturated fatty acid-containing oil, tackifier, sweetener, lactic acid mixture, flavoring agent, preservative, and emulsifier.

Korea Patent publication No. 10-1997-0025405 A (Prior art 4) discloses a Lactobacillus preparations of which Lactobacillus are not killed by stomach acid and show medicinal activity in the small and large intestine, and the microcapsule preparation of lactic acid bacteria is prepared by the process as follows: apart from the mixture of fat and emulsifier warmed to about 60 °C, the two mixed solutions of the protective agent and lactic acid bacteria mixed at about 50 °C is warmed and emulsified at about 60 °C, and the emulsification solution is sprayed under high pressure in the cooled dispersion at 4 °C to afford microcapsule preparation of lactic acid bacteria, of which settlement rate of lactic acid bacteria in the intestine increases by preventing the death of lactic acid bacteria by stomach acid, resulting in being effective in improving intestinal health.

Korea Patent publication No. 10-2023-0001175 A (Prior art 5) discloses a complex functionalized enteric-coated soft capsule that contains both high content of lactic acid bacteria and edible oils, while guaranteeing the number of lactic acid bacteria during the shelf life, specifically, a lactic acid bacteria complex for enteric coated soft capsule containing lactic acid bacteria powder 5.0 ~ 25.0 wt%, suspension agent 5.0 ~ 20.0 wt%, emulsifier 0.5 ~ 2.0 wt% and residual edible oil.

Korea Patent publication No. 10-2021-0083423 A (Prior art 6) discloses an oral paste containing about 80-90% oil, about 0.5-20% emulsifier, and about 1-2% probiotic lactic acid bacteria effective against tartar and plaque.
However, there has been not reported or disclosed about the gastric acid-stable probiotic formulation containing edible oils, tree nuts, and emulsifiers and the method thereof than those in the above cited literatures, the disclosures of which are incorporated herein by reference.

Accordingly, the present inventors have conducted researches to develop novel lactic acid bacteria formulation that is superior to the previous lactic acid bacteria complex containing lactic acid bacteria, edible oils, fats and emulsifiers disclosed in the existing Prior Patent No. 5 (Korea Patent publication No. 10-2023-0001175 A (Prior art 5)).

It can be confirmed that the lactic acid bacteria preparation of the present invention strongly exerts an excellent and advanced effect than the previously known lactic acid bacteria formulation through various experiments, for examples, (1) experiment for the preparation of emulsion by heating (Experimental Example 1); (2) Effect on the proliferation of beneficial bacteria in the intestine (Experimental Example 2); (3) Lactobacillus viability experiment in artificial gastric juice (pH 2) (Experimental Example 3); (4) Efficacy test of composition for improving constipation syndrome (simple clinical test 1); (5) palatability evaluation experiment according to the type of nuts contained in the composition (simple clinical test 2), and found the inventive composition strongly proliferates beneficial bacteria in the intestine, exhibits excellent viability of lactic acid bacteria in stomach acid, and exerts an excellent and advanced effect in terms of improvement of constipation and taste than the previously known lactic acid bacteria preparation.

At the result, it has been confirmed that the present invention can provides excellent probiotics and finally, completed the present invention.

### SUMMARY OF THE INVENTION

The present invention was developed to overcome the above problems. The present invention is a gastric acid-stable probiotic formulation comprising lactic acid bacteria, edible oils, emulsifiers, and nuts, and it is intended to provide an excellent probiotic formulation that strongly proliferate beneficial bacteria in the intestine, exhibit excellent viability in gastric acid, and exert superior and advanced efficacy than previously known lactobacillus preparations in terms of improvement of constipation and palatability and the preparation method thereof

The characteristic constitution of the present invention in order to achieve the purpose of the present invention as described above and to realize the characteristic effect of the present invention described below is as follows.

As a preferred embodiment of the present invention, the present invention provides a gastric acid-stable probiotic formulation, which is prepared by the process comprising the steps of (1) heating the edible oil 1 to 90 weight part to 70 to 95 °C and then mixing and stirring the emulsifier to 0.1 to 5 weight part to obtain the mixture of an edible oil and emulsifier at the first step; cooling the mixture and stirring until the temperature is between 2 and 20°C, to obtain a gel-like flocculating mixture at the second stage; slowly injecting 1 weight part of lactic acid bacteria into the gel-shaped flocculation mixture with low-speed stirring to obtain lactic acid bacteria mixture at the third step.

As a further preferred embodiment of the present invention, the present invention provides additional further steps comprising the steps of adding a crushed nut material to the lactic acid bacteria mixture to obtain the nut and lactic acid bacteria mixture at the fourth step; and optionally, adding one or more additional ingredients randomly selected from prebiotics and cohesive agents to provide an inventive gastric acid-stable probiotic formulation.

As a preferred embodiment of the present invention, the above described nut crushed material has a particle size of 0.01 to 0.5 mm, and it is prepared by stirring the crushed nut material at the speed of 30 to 50 rpm to the lactic acid bacteria mixture to obtain the nut and lactic acid bacteria mixture, until all ingredients are evenly mixed for 30 minutes to 3 hours.

As a preferred embodiment of the present invention, the inventive gastric acid-stable probiotic formulation contains (a) lactic acid bacteria, (b) edible oils, (c) emulsifiers and (d) nuts as essential ingredients.

As a preferred embodiment of the present invention, the relative weight ratio of the individual components of the inventive gastric acid-stable probiotic formulation consists of (a) lactic acid bacteria 1.0 weight part, (b) edible oil 1 ~ 90 weight part, (c) emulsifier 0.1 ~ 5.0 weight part and (d) nuts 5.0 ~ 90.0 weight part.

As a preferred embodiment of the present invention, the term "(a) lactic acid bacteria", as defined herein, is characterized by one or more strains selected from microorganisms consisting of the genus Lactobacillus, Bifidobacterium, Bacillus, Streptococcus, and Enterococcus.

As a desirable embodiment of the present invention, the term " (b) edible oils", as defined herein, are characterized by one or more ingredients selected from a group of avocado oil, canola oil, evening primrose oil, coconut oil, Brazil nut oil, corn oil, cottonseed oil, flaxseed oil, grapeseed oil, hemp oil, olive oil, palm oil, safflower seed oil, soybean oil, peanut oil, sunflower seed oil, krill oil, anchovy oil, salmon oil, rice bran oil, brown rice oil, virgin coconut oil, rosehip oil, and tuna oil.

As a preferred embodiment of the present invention, the term " (c) emulsifiers", as defined herein, are characterized by one or more components selected from a group consisting of propylene glycol fatty acid esters, glycerin fatty acid esters, sucrose fatty acid esters, polyglycerin fatty acid esters, sorbitan fatty acid esters, organic acid monoglycerides, polyoxyethylene fatty acid esters, propylene glycol fatty acid esters, polyoxyethylene fatty acid esters, polysorbate, egg yolk lecithin, soybean lecithin, carboxymethylcellulose, glycerin, soy phospholipids, and stearyl calcium lactate.

As a preferred embodiment of the present invention, the term "(d) nuts", as defined herein is characterized by one or more components selected from a group of peanuts, almonds, walnuts, pine nuts, pistachios, pecans, macadamias, hazelnuts, cashews, Brazil nuts, chestnuts, soybeans, cacao nibs, coconuts, sunflower seeds, and pumpkin seeds.

As a preferred embodiment of the present invention, the inventive gastric acid-stable probiotic formulation comprises one or more additional ingredients selected from (e) prebiotics, and (f) cohesive agents in addition to the essential ingredients consisting of (a) lactic acid bacteria, (b) edible oils, (c) emulsifiers and (d) nuts.

As a preferred embodiment of the present invention, the relative weight ratio of the individual components of a gastric acid-stable probiotic formulation consists of (a) lactic acid bacteria 1.0 weight part, (b) edible oil 1 ~ 60 weight part, (c) emulsifier 0.1 ~ 5.0 weight part, (d) nuts 5.0 ~ 90.0 weight part, (e) prebiotics 0.01 ~ 5.0 weight part, and (f) cohesive agents 0.1 ~ 5.0 weight part.

As a preferred embodiment of the present invention, the term "(e) prebiotics", as defined herein, is characterized by being one or more components selected from pentose such as xylose and arabinose, hexose such as glucose, mannose, fructose, and galactose; lactulose, lactitol, sucrose, lactose, maltose, trehalose; fructo-oligosaccharide, raffinose, stachyose, maltodextrin, amylose, amylopectin, starch, cellulose, and pectin.

As a preferred embodiment of the present invention, "(f) the cohesive agents", as defined herein, is characterized by one or more components selected from a group consisting of sodium silico aluminate, xanthan gum, carrageenan, guar gum, diutan gum, cellulose gum, gellan gum, pectin, and carboxymethylcellulose.

Hereinafter, in order to facilitate the implementation of the present invention by a person with ordinary knowledge in the art, the desirable embodiments of the present invention shall be described in detail by referring to the attached drawings.

The inventive gastric acid-stable probiotic formulation according to the present invention is prepared by the process comprising the steps of (1) heating the edible oil 1 to 90 weight part to 70 to 95 °C, preferably 75 to 85 °C, and then mixing and stirring the emulsifier to 0.1 to 5 weight part to obtain the mixture of an edible oil and emulsifier at the first step; cooling the mixture and stirring until the temperature is between 2 and 20°C, preferably 4 to 10 °C, to obtain a gel-like flocculating mixture at the second stage; slowly injecting 1 weight part of lactic acid bacteria into the gel-shaped flocculation mixture with low-speed stirring to obtain lactic acid bacteria mixture at the third step.
accordingly, the present invention also provides a method for preparing the inventive gastric acid-stable probiotic formulation according to the present invention, comprising the steps of (1) heating the edible oil 1 to 90 weight part to 70 to 95 °C, preferably 75 to 85 °C, and then mixing and stirring the emulsifier to 0.1 to 5 weight part to obtain the mixture of an edible oil and emulsifier at the first step; cooling the mixture and stirring until the temperature is between 2 and 20°C, preferably 4 to 10 °C, to obtain a gel-like flocculating mixture at the second stage; slowly injecting 1 weight part of lactic acid bacteria into the gel-shaped flocculation mixture with low-speed stirring to obtain lactic acid bacteria mixture at the third step.

Through this process, the emulsifier is dissolved in the heated edible oil, thoroughly mixed, and then cooled to produce a stable gel-like mixture, and then mixed with lactic acid bacteria, which can excellently improve the intestinal retention of lactic acid bacteria.

As a further preferred embodiment of the present invention, the present invention provides additional further steps comprising the steps of adding a crushed nut material to the lactic acid bacteria mixture prepared at 3^{rd} step to obtain the nut and lactic acid bacteria mixture at the fourth step; and optionally, adding one or more additional ingredients randomly selected from prebiotics and cohesive agents at the fifth step to provide an inventive gastric acid-stable probiotic formulation.

As a preferred embodiment of the present invention, the above described nut crushed material has a particle size of 0.01 to 0.5 mm, preferably, 0.01 to 0. 1mm, and it is prepared by stirring the crushed nut material at the speed of 30 to 50 rpm to the lactic acid bacteria mixture to obtain the nut and lactic acid bacteria mixture, until all ingredients are evenly mixed for 30 minutes to 3 hours.

As a preferred embodiment of the present invention, the inventive probiotic formulation prepared in the fifth step may be sealed under nitrogen gas and stored at 25°C or below. The inventive probiotic formulation can be controlled below 30°C and the deterioration of lactic acid bacteria content due to temperature and physical friction can be minimized through low-speed stirring process.

The term "(a) lactic acid bacteria" defined herein means a strain that helps digestion and absorption and inhibits the growth of harmful bacteria in the intestine so that the intestinal environment is in a desirable state, and the form of lactic acid bacteria is not limited thereto, but various forms such as dry powder and dried coarse are preferable, and the size of the strain may be in the range of 0.1 ~ 8.0 µm, preferably 0.2 ~ 4.0 µm.

In the present invention, the content of individual constituent components is to be expressed as a relative weight part based on the lactic acid bacteria 1 weight part.

The representative strain of the present invention is preferably a random lactic acid bacterium that is widely used as a probiotic, and is readily available in the technical field, such as the American Type Culture Collection (ATCC), the Korean Collection for Type Cultures (KCTC), the Korean Culture Center of Microorganisms (KCCM) affiliated with the Korea Seed Bacteria Association, and the Korean Agricultural Culture Collection (KACC) of the Korean Rural Development Administration.

Specifically, the lactic acid bacterium belonged to genus of Lactobacillus, Bifidobacterium, Bacillus, Streptococcus, or Enterococcus genus; preferably, Lactobacillus, Bifidobacterium, or Streptococcus genus, more preferably, Bifidobacterium, or Streptococcus genus; more specifically, Bifidobacterium such as Bifidobacterium longum, Bifidobacterium bifidum, Bifidobacterium breve, Bifidobacterium animalis ssp. lactis, Bifidobacterium adolescentis, Bifidobacterium pseudocatenulatum, Bifidobacterium catenulatum or Bifidobacterium infantis, B. thermophilum species etc;
Lactobacillus species such as Lactobacillus plantarum, Lactobacillus pentosus, Lactobacillus casei, Lactobacillus casei ssp. paracasei, Lactobacillus rhamnosus, Lactobacillus acidophilus, Lactobacillus delbrueckii, Lactobacillus delbrueckii ssp. bulgaricus, Lactobacillus delbrueckii ssp. delbrueckii, Lactobacillus fermentum, Lactobacillus gasseri, Lactobacillus reuteri, Lactobacillus brevis, Lactobacillus cellobiosus, Lactobacillus crispatus, Lactobacillus GG, Lactobacillus johnsonii, Lactobacillus lactis, Lactobacillus reuteri, Lactobacillus Salivarius etc;
Bacillus species such as Bacillus cereus toyoi or Bacillus cereus etc;
Leuconostoc species such as Leuconostoc citreum, Leuconostoc mesenteroides etc.
Pediococcus species such as Pediococcus acidilactici, Pediococcus pentosaceus etc; Propionibacterium species such as Propionibacterium acidifaciens, Propionibacterium acidipropionici etc;
Streptococcus species such as Streptococcus thermophilus, Streptococcus cremoris, Streptococcus infantarius, Streptococcus intermedius, Streptococcus lactis, Streptococcus salivarius subsp. etc;
Enterococcus species such as Enterococcus faecalis or Enterococcus faecium etc;
Saccharomyces species such as Saccharomyces cerevisiae, Saccharomyces boulardii etc;
More specifically, Lactobacillus or Bifidobacterium genus strain, more preferably, at least one or two strains selected from the group consisting of Lactobacillus acidophilus, Lactobacillus rhamnosus GG, Lactobacillus plantarum, Bifidobacterium longum, Bifidobacterium animalis ssp. Lactis;
More and more preferably, at least one or two strains selected from the group consisting of Lactobacillus acidophilus, Lactobacillus rhamnosus GG, Lactobacillus plantarum, Bifidobacterium longum and Bifidobacterium animalis ssp. Lactis.

The term " (b) edible oils", as defined herein, are characterized by one or more ingredients selected from a group of avocado oil, canola oil, evening primrose oil, coconut oil, Brazil nut oil, corn oil, cottonseed oil, flaxseed oil, grapeseed oil, hemp oil, olive oil, palm oil, safflower seed oil, soybean oil, peanut oil, sunflower seed oil, krill oil, anchovy oil, salmon oil, rice bran oil, brown rice oil, coconut oil, rosehip oil, and tuna oil.

The edible oil is to protect against stomach acid by wrapping the lactic acid bacteria complex. Edible oils are mixed with 1 to 90 weight parts relative to 1 weight part of lactic acid bacteria, preferably mixed with 15 to 30 weight parts.

First of all, the edible oils are heated to 70 to 95 °C, preferably to 75 to 85 °C, and then the heated edible oil is stirred with an emulsifier until the temperature of the mixture is reached to 2 to 20 °C, preferably 4 to 10 °C, in a circulating cooling water bath maintained at 0 to 10 °C, preferably 0 to 5 °C, to form a gel-like stable flocculating mixture.

The term " (c) emulsifiers", as defined herein, are characterized by one or more components selected from a group consisting of propylene glycol fatty acid esters, glycerin fatty acid esters, sucrose fatty acid esters, polyglycerin fatty acid esters, sorbitan fatty acid esters, organic acid monoglycerides, polyoxyethylene fatty acid esters, propylene glycol fatty acid esters, polyoxyethylene fatty acid esters, polysorbate, egg yolk lecithin, soybean lecithin, carboxymethylcellulose, glycerin, soy phospholipids, and stearyl calcium lactate.

The emulsifier is to play a role in increasing the miscibility between the compositions in the lactic acid bacteria complex, and after heating the edible oil to 70 to 95 °C, preferably 75 to 85 °C, when the emulsifier is mixed, the emulsifier is melted, and the mixability with edible oils is greatly increased.

Conventionally, the process of mixing the emulsifier by heating the edible oil to 60°C has been known, but there has been some needs to dissolve them thoroughly since the emulsifier could not mixed with the edible oil thoroughly although the edible oil and emulsifier are heated to 60°C.

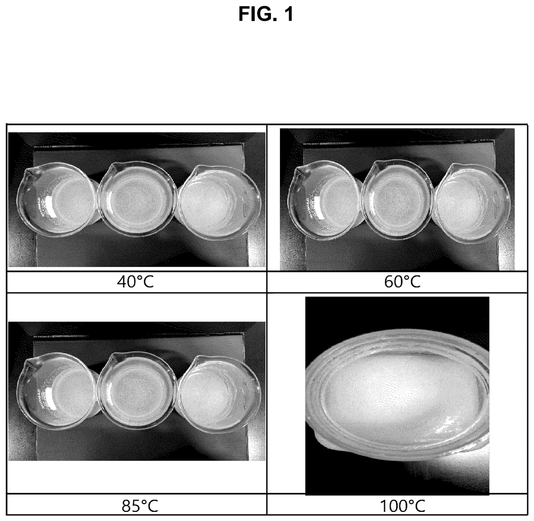

The present inventors have found that been that the edible oil shall be heated to at least 70°C in order to dissolving the emulsifier in the edible oil, and if the edible oil is heated to more than 100°C, there is a possibility of acidification.. Therefore, it has been confirmed that it is preferable to heat the edible oil to a temperature of 75 to 85 ° C to dissolve an emulsifier (See Fig. 1)

The content of emulsifier can be included in the 0.1 ~ 5.0 weight part, preferably, 0.20 ~ 1.60 weight part, more preferably 0.30 ~ 1 weight part based on lactic acid bacteria1 weight part.

At this time, if the emulsifier content is less than 0.1 weight part, there may be some problem of poor miscibility with lactic acid bacteria mixed powder and edible oil, and even if it is used in excess of 5.0 weight part, there is no longer an increase in miscibility between compositions, so it is appropriate to use it within the above range. As a preferred embodiment of the present invention, the inventive probiotic formulation contains (a) lactic acid bacteria, (b) edible oils, (c) emulsifiers and (d) nuts as essential ingredients.

As a preferred embodiment of the present invention, the relative weight ratio of the individual components of the inventive probiotic formulation consists of (a) lactic acid bacteria 1.0 weight part, (b) edible oil 1 ~ 90 weight part, (c) emulsifier 0.1 ~ 5.0 weight part and (d) nuts 5.0 ~ 90 weight part.

Among these, the detailed descriptions on (a) lactic acid bacteria, (b) edible oils and fats, and (c) emulsifiers are omitted as they have been fully detailed above.

As a preferred embodiment of the present invention, the term " (d) nuts", as defined herein is characterized by one or more components selected from a group of peanuts, almonds, walnuts, pine nuts, pistachios, pecans, macadamias, hazelnuts, cashews, Brazil nuts, chestnuts, soybeans, cacao nibs, coconuts, sunflower seeds, and pumpkin seeds as an additive to form a suitable viscosity of the composition, to increase the flavor/texture, and further to enhance the activity of lactic acid bacteria.

The nuts containing a large amount of unsaturated fatty acids, are essential ingredients that play a beneficial role in various healthy effect including blood circulation improvement, and the nuts are preferable in the form of a crushed nut material, and the suitable particle size of the crushed nut material is 0.01 to 0.5 mm, preferably 0.01 to 0.1 mm to the extent that it shows a paste shape.

The content of nuts is mixed in the 5.0 to 90.0 weight part based on lactic acid bacteria 1 weight part, preferably 10.0 to 30.0 weight based on lactic acid bacteria 1 weight part.

The present inventors have found that nuts not only improve the palatability and organolepcy of probiotic formulation, but also have an excellent effect on maintaining the viability of lactic acid bacteria in stomach acid through several experiments.

As a preferred embodiment of the present invention, the inventive gastric acid-stable probiotic formulation comprises one or more additional ingredients selected from (e) prebiotics, and (f) cohesive agents in addition to the above essential ingredients.

As a preferred embodiment of the present invention, the relative weight ratio of the individual components of gastric acid-stable probiotic formulation consists of (a) lactic acid bacteria 1.0 weight part, (b) edible oil 1 ~ 90 weight part, (c) emulsifier 0.1 ~ 5.0 weight part, (d) nuts 5.0 ~ 90.0 weight part, (e) prebiotics 0.01 ~ 5.0 weight part, and (f) cohesive agents 0.1 ~ 5.0 weight part; more preferably, (a) lactic acid bacteria 1.0 weight part, (b) edible oil 10 ~ 30 weight part, (c) emulsifier 0.2 ~ 1.6 weight part, (d) nuts 15.0 ~ 30.0 weight part, (e) prebiotics 0.5 ~ 2.0 weight part, and (f) cohesive agents 2.0 ~ 5.0 weight part.

The term, "(e) prebiotics" defined herein are used as food for lactic acid bacteria, and are not digested by the digestive tract enzymes of animals, but proliferate lactic acid bacteria such as bifidobacteria, which are intestinal bacteria, in the lower part of the small intestine, and exhibit various physiological effects including intestinal action, which comprise monosaccharides, disaccharides, oligosaccharides, and polysaccharides etc as well-known prebiotics in the sugar industry, preferably pentose such as xylose and arabinose etc; hexose such as glucose, mannose, fructose, and galactose; disaccharides such as lactulose, lactitol, sugar, lactose, maltose, and trehalose; oligosaccharides, such as fructo-oligosaccharides, raffinose, stachyose, and maltodextrin; polysaccharides such as amylose, amylopectin, starch, cellulose, and pectin; more preferably, glucose, fructose, galactose, sucrose, lactose, fructo-oligosaccharide, etc., more and more preferably, glucose, fructose, lactulose, lactitol, or fructo-oligosaccharides.

The content of (e) prebiotics can be added or reduced to a dose well-known in the art according to the type and content of (a) lactic acid bacteria, and it is desirable to put 0.01 to 5 weight parts, preferably 0.5 to 2.0 weight parts, based on lactic acid bacteria 1 weight part.

The term, "(f) the cohesive agents", as defined herein contains 0.1 to 5 weight parts, compared to 1 weight of lactic acid bacteria, and serves to give viscosity to the composition, which is preferably one or more components selected from a group consisting of sodium silico aluminate, xanthan gum, carrageenan, guar gum, diutan gum, cellulose gum, gellan gum, pectin, and carboxymethylcellulose.

If the content of the cohesive agents is less than 0.1 weight part, the effect is insignificant, and if the content of the cohesive agents exceeds 5 weight part, the viscosity may increase excessively and the workability may decrease.

In addition, the present invention can additionally add one or more additives selected in the appropriate amount of color, fragrance, etc., to the composition in the range of about 0 ~ 10 weight parts compared to the total lactic acid bacteria 1 weight part.

Accordingly, it is an object of the present invention to provide a pharmaceutical composition, a health functional food, a health care food, a dietary supplement or food additives comprising the gastric acid-stable probiotic formulation prepared by the above-described process for promoting the proliferation of beneficial bacteria to treat or prevent the disease caused by the proliferation of harmful bacteria in the intestine.

The present inventors have confirmed that the lactic acid bacteria formulation of the present invention strongly exerts an excellent and advanced effect than the previously known lactic acid bacteria preparation through various experiments, for examples, (1) experiment for the preparation of emulsion by heating temperature (Experimental Example 1); (2) Effect on the proliferation of beneficial bacteria in the intestine (Experimental Example 2); (3) Lactobacillus viability experiment in artificial gastric juice (pH 2) (Experimental Example 3); (4) Efficacy test of composition for improving constipation syndrome (simple clinical test 1); (5) palatability evaluation experiment according to the type of nuts contained in the composition (simple clinical test 2), and found the inventive composition strongly proliferates beneficial bacteria in the intestine, exhibits excellent viability of lactic acid bacteria in stomach acid, and exerts an excellent and advanced effect in terms of improvement of constipation and taste than the previously known lactic acid bacteria preparation.

In order to solve the above purpose, the present invention provide a pharmaceutical composition comprising the inventive probiotic formulation as an active ingredient to treat or prevent the disease caused by the proliferation of harmful bacteria in the intestine.

The term, "the disease caused by the proliferation of harmful bacteria in the intestine", as defined herein includes an antibiotic-associated diarrhea, ulcerative colitis, inflammatory bowel diseases such as Crohn's disease, bloating, constipation, acute or chronic irritable colitis, radiation-induced enterocolitis, and Helicobacter infections etc.

The inventive composition may comprise the above probiotic formulation as 0.01 ~ 99%, by weight based on the total weight of the composition.

However, the above composition is not necessarily limited to thereto, but may change depending on the patient's condition and the type and progression of the disease.

The composition according to the present invention can be provided as a pharmaceutical composition containing pharmaceutically acceptable carriers, adjuvants or diluents.

Pharmaceutical preparations containing present composition may be prepared in any form, such as oral dosage form (powder, tablet, capsule, soft capsule, aqueous medicine, syrup, elixirs pill, powder, sachet, granule), or topical preparation (cream, ointment, lotion, gel, balm, patch, paste, spray solution, aerosol and the like), or injectable preparation (solution, suspension, emulsion).

Pharmaceutically acceptable carriers, adjuvants or diluents such as lactose, dextrose, sucrose, sorbitol, mannitol, xylitol, erythritol, maltitol, starches, acacia rubber, alginate, gelatin, calcium phosphate, calcium silicate, cellulose, methyl cellulose, polyvinyl pyrrolidone, water, methylhydroxy benzoate, propylhydroxy benzoate, magnesium stearate and mineral oil etc may be used herein.

The preparations may additionally include fillers, anti-agglutinating agents, lubricating agents, wetting agents, flavoring agents, emulsifiers, preservatives and the like.

In the case of preparations, it is prepared using diluents or excipients such as fillers, extenders, binders, humectants, disintegrators, surfactants, etc.

Solid preparations for oral administration include tablets, pills, powders, granules, capsules, etc., and these solid formulation are prepared by mixing the inventive formulation with at least one excipient selected from starch, calcium carbonate, sucrose or lactose, gelatin, etc. In addition to simple excipients, lubricants such as magnesium stearate, talc are also used.

Liquid type oral preparations of the present invention include suspensions, liquids, emulsions, syrups, etc., and may include various excipients, such as humectants, sweeteners, fragrances, and preservatives in addition to water and liquid paraffin, which are commonly used simple thinners.

Preparations for parenteral administration include sterile aqueous solutions, non-aqueous solvents, suspensions, emulsions, lyophilized preparations, and suppositories. Non-aqueous solvents and suspensions may include olive oil such as propylene glycol, polyethylene glycol, vegetable oils and injectable esters such as ethyl oleate. Suppositories such as witepsol, macrogol, tween 61, cacao, lauringe, glycerozeratin, etc. may be used.

The desirable dose of the inventive probiotic formulation varies depending on the condition and the weight of the subject, severity, drug form, route and period of administration, and may be chosen by those skilled in the art. However, in order to obtain desirable effects, it is generally recommended to administer at the amount ranging from 0.01 to 10g/kg preferably, 0.0mg/kg to 1g/kg by weight/day of the inventive probiotic formulation of the present invention. The dose may be administered in single or divided into several times per day. Therefore, the above dosage does not in any way limit the scope of the invention.

The pharmaceutical composition of present invention can be administered to a subject animal such as mammals (rat, mouse, domestic animals or human) *via* various routes. All modes of administration are contemplated, for example, administration can be made orally, rectally or by intravenous, intramuscular, subcutaneous injection, etc.

In accordance with another aspect of the present invention, there is also provided a method of treating or preventing the disease caused by the proliferation of harmful bacteria in the intestine in mammals, wherein the method comprises administering a composition comprising a therapeutically effective amount inventive probiotic formulation, into the mammal suffering from the disease caused by the proliferation of harmful bacteria in the intestine.

In accordance with another aspect of the present invention, there is also provided a use of a composition comprising inventive probiotic formulation, for manufacture of medicines employed for treating or preventing the disease caused by the proliferation of harmful bacteria in the intestine.

Accordingly, it is the other object of the present invention to provide a health functional food comprising a therapeutically effective amount of the inventive probiotic formulation for the prevention or alleviation of the disease caused by the proliferation of harmful bacteria in the intestine.

The inventive composition may comprise above probiotic formulation as 0.01 ~ 99%, preferably, 0.1 ~ 50% by weight based on the total weight of the composition.

However, the above composition is not necessarily limited to thereto, but may change depending on the patient's condition and the type and progression of the disease.

The composition according to the present invention can be provided as a pharmaceutical composition containing pharmaceutically acceptable carriers, adjuvants or diluents.

Pharmaceutical preparations containing present composition may be prepared in any form, such as oral dosage form (powder, tablet, capsule, soft capsule, aqueous medicine, syrup, elixirs pill, powder, sachet, granule), or topical preparation (cream, ointment, lotion, gel, balm, patch, paste, spray solution, aerosol and the like), or injectable preparation (solution, suspension, emulsion).

Pharmaceutically acceptable carriers, adjuvants or diluents such as lactose, dextrose, sucrose, sorbitol, mannitol, xylitol, erythritol, maltitol, starches, acacia rubber, alginate, gelatin, calcium phosphate, calcium silicate, cellulose, methyl cellulose, polyvinyl pyrrolidone, water, methylhydroxy benzoate, propylhydroxy benzoate, magnesium stearate and mineral oil etc may be used herein.

The preparations may additionally include fillers, anti-agglutinating agents, lubricating agents, wetting agents, flavoring agents, emulsifiers, preservatives and the like.

In the case of preparations, it is prepared using diluents or excipients such as fillers, extenders, binders, humectants, disintegrators, surfactants, etc.

Solid preparations for oral administration include tablets, pills, powders, granules, capsules, etc., and these solid formulation are prepared by mixing the inventive formulation with at least one excipient selected from starch, calcium carbonate, sucrose or lactose, gelatin, etc. In addition to simple excipients, lubricants such as magnesium stearate, talc are also used.

Liquid type oral preparations of the present invention include suspensions, liquids, emulsions, syrups, etc., and may include various excipients, such as humectants, sweeteners, fragrances, and preservatives in addition to water and liquid paraffin, which are commonly used simple thinners.
. Preparations for parenteral administration include sterile aqueous solutions, non-aqueous solvents, suspensions, emulsions, lyophilized preparations, and suppositories. Non-aqueous solvents and suspensions may include olive oil such as propylene glycol, polyethylene glycol, vegetable oils and injectable esters such as ethyl oleate. Suppositories such as witepsol, macrogol, tween 61, cacao, lauringe, glycerogelatin, etc. may be used.

The desirable dose of the inventive probiotic formulation varies depending on the condition and the weight of the subject, severity, drug form, route and period of administration, and may be chosen by those skilled in the art. However, in order to obtain desirable effects, it is generally recommended to administer at the amount ranging from 0.01 mg/kgto 10g/kg preferably, 1.0mg/kg to 1g/kg by weight/day of the inventive probiotic formulation of the present invention. The dose may be administered in single or divided into several times per day. Therefore, the above dosage does not in any way limit the scope of the invention.

Accordingly, it is the other object of the present invention to provide a health functional food comprising a therapeutically effective amount of the inventive probiotic formulation for promoting the proliferation of beneficial bacteria and for the prevention or alleviation of the disease caused by the proliferation of harmful bacteria in the intestine.The inventive composition may comprises above probiotic formulation as 0.01 ~ 99%, preferably, 0.1 ~ 50% by weight based on the total weight of the composition.

The term "a functional health food" defined herein" the functional food having enhanced functionality such as physical functionality or physiological functionality by adding the inventive formulation of the present invention to conventional food to prevent or improve the purposed diseases in human or mammal, which is stipulated by Korean functional health food Law No.6727.

The inventive functional health food may comprises above probiotic formulation as 0.01 ~ 95%, preferably, 1 ~ 80% by weight based on the total weight of the composition.

Furthermore, the composition of the present invention may be a health care food for the purpose of treating or improving the disease caused by the proliferation of harmful bacteria in the intestine.

In addition, for the purpose of preventing and improving the disease caused by the proliferation of harmful bacteria in the intestine, it can be manufactured and processed as health functional foods in the form of pharmaceutical administrations such as acids, granules, tablets, capsules, pills, suspensions, emulsions, syrups, etc., or in the form of tea agents, leached teas, and health drinks etc.

Accordingly, it is the other object of the present invention to provide a health care food or diet supplement comprising an inventive probiotic formulation for the prevention or alleviation of the disease caused by the proliferation of harmful bacteria in the intestine.

The main ingredient in a health care food as defined herein means that the content of the active ingredient relative to the weight of the total composition is in the range of approximately 30% to 99%, preferably in the range of 50% to 99%, and preferably in the range of 70% to 99%.

Providing that the health beverage composition of present invention contains above described formulation(s) as an essential component in the indicated ratio, there is no particular limitation on the other liquid component, wherein the other component can be various deodorant or natural carbohydrate etc such as conventional beverage. Examples of aforementioned natural carbohydrate are monosaccharide such as glucose, fructose etc; disaccharide such as maltose, sucrose etc; conventional sugar such as dextrin, cyclodextrin; and sugar alcohol such as xylitol, and erythritol etc. As the other deodorant than aforementioned ones, natural deodorant such as taumatin, stevia extract such as levaudiosideA, glycyrrhizin et al., and synthetic deodorant such as saccharin, aspartam et al., may be useful favorably. The amount of above described natural carbohydrate is generally ranges from about 0 to 20 g in the ratio of 100 mℓ of present beverage composition.

In addition, the probiotic preparation of the present invention may be added to food or beverage for the purpose of preventing hangover or liver disease. At this time, the amount of the probiotic formulation in food or beverage can be added from 0.01 to 15% by weight of the total food weight, and the health drink composition can be added in a ratio of 0.02 to 5 g, preferably 0.3 to 1 g based on 100 ml.

Accordingly, it is the other object of the present invention to provide a food or food additive comprising an inventive gastric acid-stable probiotic formulation for the prevention or alleviation of the disease caused by the proliferation of harmful bacteria in the intestine.

The items listed in the "Food Additives Code" include, for example, chemically synthesized products such as ketones, glycine, potassium citrate, nicotinic acid, cinnamic acid, persimmon coloring, licorice extract, crystalline cellulose, guar gum, etc., mixed preparations such as L-monosodium glutamate, noodle additive alkaline agents, preservatives, and tar pigment preparations.

Functional health foods containing probiotic preparations of the present invention include bread, rice cakes, dried fruits, candy, chocolates, chewing gum, confectionery such as jams, ice cream, ice cream, ice cream products, ice cream products, milk products, low-fat milk, lactose-lyzed milk, processed milk, goat milk, fermented milk, butter oil, concentrated milk, milk cream, natural cheese, processed cheese, milk powder, processed milk products such as whey, processed meat products, processed egg products, meat products such as hamburgers, fish meat products such as fish cakes, ham, sausage, bacon, etc. Noodles such as yutang noodles, luxurious dried noodles, modified noodles, frozen noodles, pasta fruits, vegetable drinks, carbonated drinks, lactic acid bacteria drinks such as soy milk, yogurt, and mixed drinks Soy sauce, soybean paste, red pepper paste, chunjang, cheonggukjang, mixed paste, vinegar, sauces, tomato ketchup, curry, seasonings such as dressing, margarine, shortening and pizza are not limited to.

In the process of manufacturing the health functional food, the content of the probiotic formulation according to the present invention added to the food including the beverage can be appropriately added or subtracted as necessary.

As described in the present invention, inventive gastric acid-stable probiotic formulation strongly exerts an excellent and advanced effect than the previously known lactic acid bacteria preparation through various experiments, for examples, (1) experiment for the preparation of emulsion by heating temperature (Experimental Example 1); (2) Effect on the proliferation of beneficial bacteria in the intestine (Experimental Example 2); (3) Lactobacillus viability experiment in artificial gastric juice (pH 2) (Experimental Example 3); (4) Efficacy test of composition for improving constipation syndrome (simple clinical test 1); (5) palatability evaluation experiment according to the type of nuts contained in the composition (simple clinical test 2), and found the inventive composition strongly proliferates beneficial bacteria in the intestine, exhibits excellent viability of lactic acid bacteria in stomach acid, and exerts an excellent and advanced effect in terms of improvement of constipation and taste than the previously known lactic acid bacteria preparation..
. Therefore, the present invention can provides the therapeutics or functional health food for treating and preventing the disease caused by the proliferation of harmful bacteria in the intestine.

### BRIEF DESCRIPTION OF THE DRAWINGS

The above and other objects, features and other advantages of the present invention will more clearly understood from the following detailed description taken in conjunction with the accompanying drawings, in which;
Fig. 1 shows the experimental results of observing the emulsion properties of the emulsifier of the present invention by various temperatures;
Fig. 2 represents the experimental results measuring the effect of strain growth on the selection medium (MRS agar plate) of the lactic acid bacteria preparation of the present invention;
Fig. 3 represents the test result of Lactobacillus viability experiment in artificial gastric juice (pH 2.).

### DETAILED DESCRIPTION OF THE INVENTION

It will be apparent to those skilled in the art that various modifications and variations can be made in the compositions, use and preparations of the present invention without departing from the spirit or scope of the invention.

The present invention is more specifically explained by the following examples. However, it should be understood that the present invention is not limited to these examples in any manner.

### EXAMPLES

The following Reference Example, Examples and Experimental Examples are intended to further illustrate the present invention without limiting its scope.

### EXAMPLE 1. Preparation of inventive emulsifier-added lactic acid bacteria formulation (Composition s : Table 1)

20g of sunflower oil (vegetable oil, Sajo Daelim Co., Ltd.) was poured into a stirring heater (Hotplate Stirrer. MSH-20D) and heated to 75 to 85 °C.

0.4 g of glycerin fatty acid ester (emulsifier, ES food ingredient) was added thereto with stirring.

The mixture of vegetable oil and emulsifier obtained by the above stage were emulsified by stirring until the temperature of the mixture was 4 to 10 °C in a circulating cooling tank (SH-WB-7CDR) maintained at 0 to 4 °C (Test-1: Preparation of emulsion solution according to various temperature, 40°C, 60°C, 85°C, 100°C and the test results refer to Experimental Example 1)

Lactobacillus acidophilus DDS-1 (Olab), Lactobacillus rhamnosus GG (Alllab), Lactobacillus plantarum VI-07 (Vitech Co., Ltd.) mixed into the emulsified oil formed in the form of a gel. Bifidobacterium longum BL5 (Vitech Co., Ltd.) and Bifidobacterium animalis (Bifidobacterium animalis ssp. lactis BLC1) were evenly mixed at the same rate (2x109cfu/g) respectively and 1 g of mixed lactic acid bacteria were used in the experiment (Test-2:, the experimental method for process the observation of the viability of lactic acid bacteria and experimental results in artificial gastric solution (pH 2) refer to the following Experimental Example 2.)

Peanuts (nuts, DaeChang Foods Co. Ltd), which contains natural sweeteners such as fructooligosaccharides (feed for lactic acid bacteria; prebiotics) and unsaturated fatty acids as additives to form suitable viscosity of the composition, increase flavor/texture, further enhance the activity of lactic acid bacteria and provides beneficial effect such as improved blood circulation etc, were added thereto and stirred with a mixing stirrer (OFM-1507B, Opel Co., Ltd.) at a speed of 30 to 50 rpm for more than 1 hour until all the ingredients were evenly mixed.

At this time, in the case of nuts, it is best to grind them with a crushing device (ultra-high-speed vacuum blender, KCB-001W, Kucharm Co., Ltd.) so that the particle size is 0.01-0.1 millimeters (mm) (the degree to which the paste shape is visible). (Test-3: the peanut was chosen among peanuts, almonds, and walnuts by the evaluation test according to various criteria such as throat clearing and palatability and test result refer to Experiment Example 3)

**[Table 1]**

| Composition of lactic acid formulation | | | |
|---|---|---|---|
| Formulation* | Composition A | Composition B | Composition C |
| Olive oil | 20 g | 20 g | 95.5 g |
| Emulsifier | 0.3 g | - | 1.5 g |
| Fructo-oligosaccharide | 1 g | 1 g | 1 g |
| crushed nut material | 76.7 g | 77 g | - |
| mixed lactic acid bacteria | 2 g | 2 g | 2 g |
| sum | 100 g | 100 g | 100 g |
| * Composition A:Olive oil+ Emulsifier+ Fructooligosaccharide + crushed nut material + mixed lactic acid bacteria / | | | |
| Composition B: Olive oil+ Fructooligosaccharide + crushed nut material + mixed lactic acid bacteria / | | | |
| Composition C: Olive oil+ Emulsifier+ Fructooligosaccharide + mixed lactic acid bacteria | | | |

### EXAMPLE 2. Preparation of emulsifier-free lactic acid bacteria formulation (Compositions: Table 1)

Composition B which does not contains the emulsifier and contains only sunflower oil (edible oil), five kinds of mixed lactic acid bacteria, and the rest of the additives (fructooligosaccharides and crushed peanut material) was prepared according to the same conditions as those described in Example 1 (Composition A) and used it as a test sample in the following Experimental Examples.

### EXAMPLE 3. Preparation of comparative lactic acid bacteria formulation

The enteric-coated lactic acid bacteria complex containing lactic acid bacteria powder 10.0 by weight, sulfur lead (suspension agent) 11.5 by weight, soy lecithin (emulsifier) 1.0 weight % and the remaining amount of rTG type refined fish oil (edible oil) disclosed in the existing literature (Korean Patent Publication No. 10-2023-0001175 A) was prepared according to the method disclaimed in Example 1 of the existing literature and used it as a comparative sample in the following experimental example.

### Experimental Example 1. Preparation of emulsion according to various heating temperature

100g of sunflower oil (vegetable oil, Sajo Daelim Co., Ltd.) was added with 2.0g of emulsifier (glycerin fatty acid ester, ES food raw material) and heated to 4 different temperatures such as 40 °C/ 60 °C/ 85 °C/ 100 °C. The mixture was stirred and emulsified until the temperature of 4 to 10 °C was measured in a circulating cooling water bath (low temperature circulation constant temperature bath, SH-WB-7CDR) maintained at 0 to 4 °C to determine the optimum temperature suitable for the preparation of emulsion.

As a result of the experiment, as shown in Figure 1, each photograph shows the shape of the emulsion formed by temperature, and at 40 °C and 60 °C, the emulsifier was not sufficiently dissolved and the emulsion was not formed properly, but at 85 °C and 100 °C, the emulsifier was sufficiently dissolved and the emulsion was evenly formed. (See Figure 1: Emulsion Properties Experiment by Various Temperatures of Emulsifiers).

### Experimental Example 2. Effect on the proliferation of beneficial bacteria in the intestine

In order to confirm the effect on the proliferation of beneficial bacteria in the intestine of the test sample obtained from the Examples, the test was performed as follows with reference to the lactic acid bacteria test method announced in the Explanation of Microbial Test Method (published by the Food and Drug Safety Evaluation Service of the Ministry of Food and Drug Safety).

### 2-1. Experimental strains

The strain was distributed by the Korean Culture Center of Microorganisms (KCCM), and Lactobacillus acidophilus ATCC 4356 (American Type Culture Collection: ATCC) was used as the beneficial gut bacteria. Lactobacilli MRS broth (288130, BD difco, USA) was used as a proliferation medium to cultivate beneficial bacteria in the intestine, and the strain was activated by passage culture more than 3 times before being used in experiments.

### 2-2. Experimental procedure

For the measurement of the number of formed lactobacillus, the number of colonies of lactic acid bacteria was measured using a medium for measuring the MRS plate, and the number of live bacteria per mL of culture medium was calculated by multiplying the average colony by the dilution multiple. 10 mL of test sample is homogenized by adding a 9-fold dilution (sterile saline) (10⁻¹ solution). The diluted solution was added to 1 mL of test solution (10⁻¹ solution) to make 10 mL to make 10⁻² test solution, and then dilution was repeated by manipulating the same.

After inoculating each diluted test solution into Lactobacillus selection medium (MRS agar plate) at 1 (v/v)% each, it was anaerobic incubation at 37°C, for 72±3 hours, and the number of colonies was calculated by selecting the flat plate that formed 15-300 colonies per 1 plate, and multiplied the measured colony number by the dilution multiple to calculate the number of bacteria.

### 2-3. test result

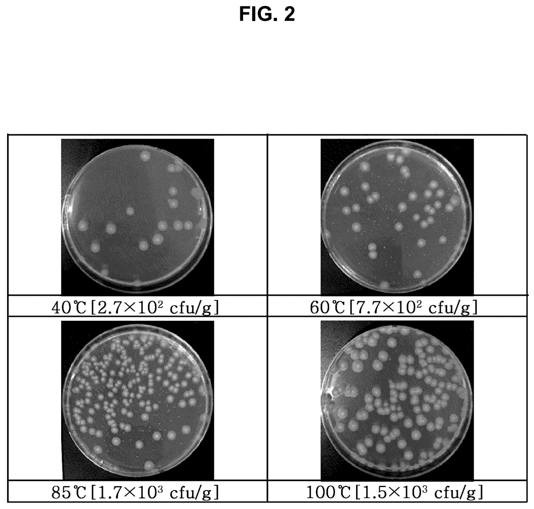

In view of the results of the viability of the test samples, it was confirmed that the emulsion solution is the best method to prepare at 85°C. (See Table 2 and Figure 2.)

**[Table 2]**

| Effect of strain growth in MRS agar plate of lactic acid bacteria formulation. | |
|---|---|
| Temperature of emulsion | Number of strains |
| 40°C | 2.7×10² cfu/g |
| 60°C | 7.7×10² cfu/g |
| 85°C | 1.7×10³ cfu/g |
| 100°C | 1.5×10³ cfu/g |

### Experimental Example 3. Effect on the viability of lactic acid bacteria in artificial gastric solution (pH 2)

In order to confirm the effect on the viability of lactic acid bacteria in artificial gastric solution (pH 2) of the sample of the present invention, the experiment was performed as follows according the method disclosed in the literature (Jeon et al., 2007, Identification and Characterization of Lactic Acid Bacteria Starters Isolated from the Commercial Drink-Yogurt Products, Korean J. Food Sci. Ani. Resour. 27(4), 509-516).

### 3-1. Experimental strains and procedure

In order to confirm the viability of lactic acid bacteria of the test samples, the composition A was stirred for a certain time (1 hour) in the artificial gastric solution (pH 2), and the collected composition was immediately cultured in a lactobacillus selection medium (MRS agar plate, MB-M1024-P50, KisanBio) to incubate at 37°C, for 72±3 hours. The flat plate that formed 15~300 colonies per 1 plate was then selected and the number of colonies was calculated.

The number of strains was calculated by multiplying the measured colony number by the dilution multiple. For comparison, the composition which un-emulsified oil was mixed with a mixture of lactic acid bacteria, was used as a control group.

### 3-2. test result

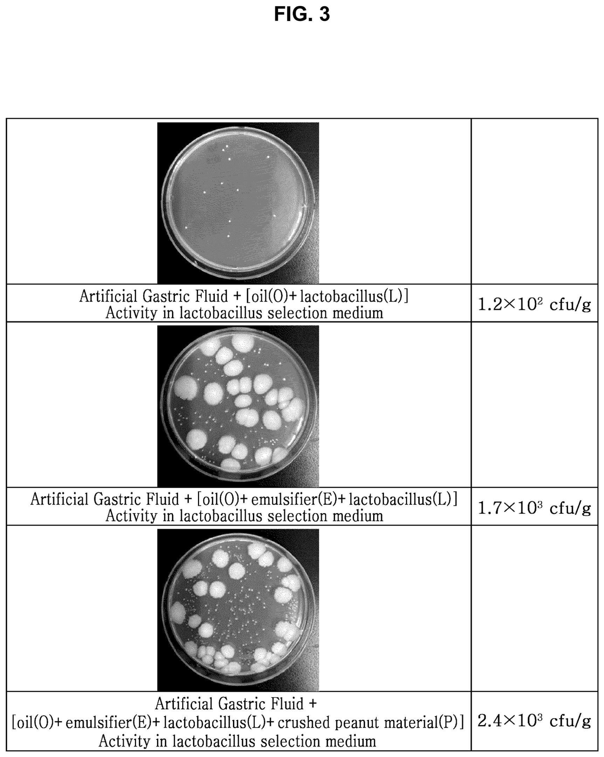

As a result of the experiment, it can be observed that the mixture of emulsified oil and lactic acid bacteria is better than the survival rate of lactobacillus in the control group. Moreover, when crushed peanut material was added to the emulsified oil as an additive and incubated under the same conditions, it has been confirmed that the survival rate of Lactobacillus in the lactobacillus mixture containing more crushed peanut material was significantly better. (See Table 3 and Fig. 3)

**[Table 3] Test Results of Lactobacillus Viability Test in Artificial Gastric Fluid (pH 2)**

| Constituent of compositions* | Number of strains |
|---|---|
| oil (O)+ lactobacillus (L) mixture | 1.2×10² cfu/g |
| oil (O) + emulsifier (E) + lactobacillus (L) mixture | 1.7×10³ cfu/g |
| oil (O)+ emulsifier (E)+ lactobacillus (L) mixture + crushed peanut material (P) | 2.4×10³ cfu/g |
| * Abbreviated: oil (O)/ emulsifier (E)/ lactobacillus (L)/ crushed peanut material (P) | |

### Simple Clinical Test 1. Efficacy test of composition for improving constipation syndrome

In order to confirm the efficacy of the sample of the present invention, the following simple clinical test was performed.

Improving efficacy of composition A on constipation, was evaluated in 50 volunteers in the Seoul area in the range of 20s to 50s who had discomfort in daily life due to constipation.

Subjects were asked to take 5 g of composition A twice a day (morning and evening) for ten days, and answered the following questions. In addition, as a control group, composition B was to be taken by 50 test subjects in the same way as the subjects

As a result of the experiment, it was confirmed that the improvement effect of composition A of the present invention on constipation is outstanding (See Table 4 and Table 5)

**[Table 4] Improving Effect of Composition A (Lactic acid bacteria composition prepared from emulsified oil) on constipation (unit: number of persons)**

| Persons(No) | Much better | A little better | No changed | |
|---|---|---|---|---|
| Defecation number | 40 | 7 | 3 | 50 |
| Time to Defecation | 35 | 12 | 3 | 50 |
| Abdominal distension | 31 | 15 | 4 | 50 |
| a sense of residual defecation | 36 | 10 | 4 | 50 |

**[Table 5] Improving Effect of Composition B (a composition of lactic acid bacteria prepared from unemulsified oils) on constipation**

| | Much better | A little better | No changed | |
|---|---|---|---|---|
| Defecation number | 11 | 26 | 13 | 50 |
| Time to Defecation | 15 | 28 | 7 | 50 |
| Abdominal distension | 7 | 21 | 22 | 50 |
| a sense of residual defecation | 16 | 23 | 11 | 50 |

### Simple Clinical Test 1. Palatability evaluation experiment according to the type of nuts contained in the composition

In order to confirm the palatability evaluation according to the type of nuts contained in the composition, the following simple clinical test was performed.

After the composition was prepared using crushed material of peanuts, almonds, and walnuts respectively, the taste, aroma, texture, and overall palatability of the nut types were evaluated in 20 adult men and women who consumed 3 g each.

The criterion for the evaluation of the overall palatability was the method of representing the mean value after the survey using the five-point scale method. (very good 5 points, good 4 points, common 3 points, bad 2 points, very bad 1 points) (Table 6)

**[Table 6] Nut Preference Evaluation Results**

| Nut type | Preference Evaluation | | | |
|---|---|---|---|---|
| | taste | aroma | texture | overall palatability |
| peanuts | 4.7 | 4.6 | 4.5 | 4.60 |
| almonds | 4.4 | 4.4 | 4.6 | 4.47 |
| walnuts | 4.1 | 3.9 | 4.6 | 4.20 |

As a result of the above experiment, it has been confirmed that the palatability of the composition using peanut powder is the best.

As described above, inventive gastric acid-stable probiotic formulation strongly exerts an excellent and advanced effect than the previously known lactic acid bacteria preparation through various experiments, for examples, (1) experiment for the preparation of emulsion by heating temperature (Experimental Example 1); (2) Effect on the proliferation of beneficial bacteria in the intestine (Experimental Example 2); (3) Lactobacillus viability experiment in artificial gastric juice (pH 2) (Experimental Example 3); (4) Efficacy test of composition for improving constipation syndrome (simple clinical test 1); (5) palatability evaluation experiment according to the type of nuts contained in the composition (simple clinical test 2), and found the inventive composition strongly proliferates beneficial bacteria in the intestine, exhibits excellent viability of lactic acid bacteria in stomach acid, and exerts an excellent and advanced effect in terms of improvement of constipation and taste than the previously known lactic acid bacteria preparation..

Hereinafter, the formulating methods and kinds of excipients will be described, but the present invention is not limited to them. The representative preparation examples were described as follows.

### Preparation of powder

Composition A 20mg
Lactose 100mg
Talc10mg

Powder preparation was prepared by mixing above components and filling sealed package.

### Preparation of tablet

Composition A 10mg
Corn Starch100mg
Lactose 100mg

### Magnesium stearate optimum amount

Tablet preparation was prepared by mixing above components and entabletting.

### Preparation of capsule

Composition A 100mg
Crystalline cellulose 3mg
Lactose 14.8mg
Magnesium stearate 0.2mg

Capsule preparation was prepared by mixing above components and filling gelatin capsule by conventional gelatin preparation method.

### Preparation of injection

Composition A 10mg
Mannitol 180mg
Distilled water for injection 2974mg
Na₂HPO₄,12H₂O 26mg

Injection preparation was prepared by filling all the components in 2mℓ ample and sterilizing by conventional injection preparation method.

### Preparation of liquid

Composition A 20mg
Sugar 10g
Mannitol 180mg
Distilled water

Liquid preparation was prepared by dissolving active component, and then filling all the components in 100 mℓ ample and sterilizing by conventional liquid preparation method.

### Preparation of health food

Composition A 1000mg
Vitamin mixture optimum amount
Vitamin A acetate 70g
Vitamin E 1.0mg
Vitamin B_{10.} 13mg
Vitamin B₂ 0.15mg
Vitamin B₆ 0.5mg
Vitamin B₁₂ 0.2g
Vitamin C 10mg
Biotin 10g
Amide nicotinic acid 1.7mg
Folic acid 50g
Calcium pantothenic acid 0.5mg
Mineral mixture optimum amount
Ferrous sulfate1.75mg
Zinc oxide 0.82mg
Magnesium carbonate 25.3mg
Monopotassium phosphate 15mg
Dicalcium phosphate 55mg
Potassium citrate 90mg
Calcium carbonate 100mg
Magnesium chloride 24.8mg

The above mentioned vitamin and mineral mixture may be varied in many ways. Such variations are not to be regarded as a departure from the spirit and scope of the present invention.

### Preparation of health beverage

Composition A 1000mg
Citric acid 1000mg
Oligosaccharide 100g
Apricot concentration 2g
Taurine1g
Distilled water 900 mℓ

Health beverage preparation was prepared by dissolving active component, mixing, stirred at 85 °C for 1 hour, filtered and then filling all the components in 1000 mℓ ample and sterilizing by conventional health beverage preparation method.

The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

As described in the present invention, inventive gastric acid-stable probiotic formulation strongly exerts an excellent and advanced effect than the previously known lactic acid bacteria preparation through various experiments, for examples, (1) experiment for the preparation of emulsion by heating temperature (Experimental Example 1); (2) Effect on the proliferation of beneficial bacteria in the intestine (Experimental Example 2); (3) Lactobacillus viability experiment in artificial gastric juice (pH 2) (Experimental Example 3); (4) Efficacy test of composition for improving constipation syndrome (simple clinical test 1); (5) palatability evaluation experiment according to the type of nuts contained in the composition (simple clinical test 2), and found the inventive composition strongly proliferates beneficial bacteria in the intestine, exhibits excellent viability of lactic acid bacteria in stomach acid, and exerts an excellent and advanced effect in terms of improvement of constipation and taste than the previously known lactic acid bacteria preparation..

## Claims

1. A method for preparing the inventive gastric acid-stable probiotic formulation, comprising the steps of (1) heating the edible oil 1 to 90 weight part to 70 to 95 °C, preferably 75 to 85 °C, and then mixing and stirring the emulsifier to 0.1 to 5 weight part to obtain the mixture of an edible oil and emulsifier at the first step; cooling the mixture and stirring until the temperature is between 2 and 20°C, preferably 4 to 10 °C, to obtain a gel-like flocculating mixture at the second stage; slowly injecting 1 weight part of lactic acid bacteria into the gel-shaped flocculation mixture with low-speed stirring to obtain lactic acid bacteria mixture at the third step.

2. The method according to claim 1,
wherein said method comprises further steps of adding a crushed nut material to the lactic acid bacteria mixture to obtain the nut and lactic acid bacteria mixture at the fourth step; and optionally, adding one or more additional ingredients randomly selected from prebiotics and cohesive agents to provide an inventive gastric acid-stable probiotic formulation.

3. The method according to claim 2,
wherein said nut crushed material has a particle size of 0.01 to 0.5 mm, and it is prepared by stirring the crushed nut material at the speed of 30 to 50 rpm to the lactic acid bacteria mixture to obtain the nut and lactic acid bacteria mixture, until all ingredients are evenly mixed for 30 minutes to 3 hours.

4. A gastric acid-stable probiotic formulation contains (a) lactic acid bacteria, (b) edible oils, (c) emulsifiers and (d) nuts as essential ingredients.

5. The gastric acid-stable probiotic formulation according to claim 4,
Wherein relative weight ratio of the individual components of the inventive gastric acid-stable probiotic formulation consists of (a) lactic acid bacteria 1.0 weight part, (b) edible oil 1 ~ 90 weight part, (c) emulsifier 0.1 ~ 5.0 weight part and (d) nuts 5.0 ~ 90.0 weight part.

6. The gastric acid-stable probiotic formulation according to claim 4,
wherein said (a) lactic acid bacteria is **characterized by** one or more strains selected from microorganisms of the genus Lactobacillus, Bifidobacterium, Bacillus, Streptococcus, and Enterococcus.

7. The gastric acid-stable probiotic formulation according to claim 4,
wherein said (b) edible oils are **characterized by** one or more ingredients selected from a group of avocado oil, canola oil, evening primrose oil, coconut oil, Brazil nut oil, corn oil, cottonseed oil, flaxseed oil, grapeseed oil, hemp oil, olive oil, palm oil, safflower seed oil, soybean oil, peanut oil, sunflower seed oil, krill oil, anchovy oil, salmon oil, rice bran oil, brown rice oil, coconut oil, rosehip oil, and tuna oil.

8. The gastric acid-stable probiotic formulation according to claim 4,
wherein said (c) emulsifiers are **characterized by** one or more components selected from a group consisting of propylene glycol fatty acid esters, glycerin fatty acid esters, sucrose fatty acid esters, polyglycerin fatty acid esters, sorbitan fatty acid esters, organic acid monoglycerides, polyoxyethylene fatty acid esters, propylene glycol fatty acid esters, polyoxyethylene fatty acid esters, polysorbate, egg yolk lecithin, soybean lecithin, carboxymethylcellulose, glycerin, soy phospholipids, and stearyl calcium lactate.

9. The gastric acid-stable probiotic formulation according to claim 4,
wherein said (d) nuts are **characterized by** one or more components selected from a group of peanuts, almonds, walnuts, pine nuts, pistachios, pecans, macadamias, hazelnuts, cashews, Brazil nuts, chestnuts, soybeans, cacao nibs, coconuts, sunflower seeds, and pumpkin seeds.

10. The gastric acid-stable probiotic formulation according to claim 4,
wherein said gastric acid-stable probiotic formulation comprises one or more additional ingredients selected from (d) nuts, (e) prebiotics, and (f) cohesive agents in addition to the essential ingredients consisting of (a) lactic acid bacteria, (b) edible oils, (c) emulsifiers and (d) nuts

11. The gastric acid-stable probiotic formulation according to claim 10,
wherein said relative weight ratio of the individual components of a gastric acid-stable probiotic formulation consists of (a) lactic acid bacteria 1.0 weight part, (b) edible oil 1 ~ 60 weight part, (c) emulsifier 0.1 ~ 5.0 weight part, (d) nuts 5.0 ~ 90.0 weight part, (e) prebiotics 0.01 ~ 5.0 weight part, and (f) cohesive agents 0.1 ~ 5.0 weight part.

12. The gastric acid-stable probiotic formulation according to claim 10,
Wherein said (e) prebiotics is **characterized by** being one or more components selected from pentose such as xylose and arabinose, hexose such as glucose, mannose, fructose, and galactose; lactulose, lactitol, sucrose, lactose, maltose, trehalose; fructo-oligosaccharide, raffinose, stachyose, maltodextrin, amylose, amylopectin, starch, cellulose, and pectin.

13. The gastric acid-stable probiotic formulation according to claim 10,
wherein said (f) the cohesive agents is **characterized by** one or more components selected from a group consisting of sodium silico aluminate, xanthan gum, carrageenan, guar gum, diutan gum, cellulose gum, gellan gum, pectin, and carboxymethylcellulose.

14. A pharmaceutical composition comprising the gastric acid-stable probiotic formulation according to claim 4 as an active ingredient to treat or prevent the disease caused by the proliferation of harmful bacteria in the intestine.

15. The pharmaceutical composition according to claim 14,
wherein the disease caused by the proliferation of harmful bacteria in the intestine includes an antibiotic-associated diarrhea, ulcerative colitis, inflammatory bowel diseases such as Crohn's disease, bloating, constipation, acute or chronic irritable colitis, radiation-induced enterocolitis, and Helicobacter infections.

16. A health functional food, comprising the gastric acid-stable probiotic formulation according to claim 4 as an active ingredient for promoting the proliferation of beneficial bacteria to improve or prevent the disease caused by the proliferation of harmful bacteria in the intestine.

17. The health functional food according to claim 16,
the health functional food is provided as powder, granule, tablet, chewing tablet, capsule or beverage type.

18. A health care food comprising the gastric acid-stable probiotic formulation according to claim 4 for the prevention or alleviation of the disease caused by the proliferation of harmful bacteria in the intestine.

19. A food additive comprising the gastric acid-stable probiotic formulation according to claim 4 for the prevention or alleviation the disease caused by the proliferation of harmful bacteria in the intestine.

20. A method of treating or preventing the disease caused by the proliferation of harmful bacteria in the intestine in mammals, wherein the method comprises administering a composition comprising a therapeutically effective amount gastric acid-stable probiotic formulation according to claim 4, into the mammal suffering from the disease caused by the proliferation of harmful bacteria in the intestine.

21. A use of a composition comprising gastric acid-stable probiotic formulation according to claim 4, for manufacture of medicines employed for treating or preventing the disease caused by the proliferation of harmful bacteria in the intestine.
